# EUROPEAN PATENT APPLICATION

(11) **EP 0 559 268 A1**
(43) Date of publication of application: **08.09.1993**
(21) Application number: 93200488.0
(22) Date of filing: 22.02.1993
(51) Int. Cl.: A61M 16/00, A61L 2/22, A61L 2/16

(54) **Apparatus and method for disinfecting a respiration apparatus**

(30) Priority: 03.03.1992 NL 9200386
(71) Applicant: PHYSIO BV, NL-2130 AR Hoofddorp (NL)
(72) Inventor: van Dijk, Geert, Maarssen (NL)
(74) Representative: Koomen, M.J.I.

(57) **Abstract**

Device and method for the disinfection of the line system of a respirating apparatus for patients, more in particular of a respirating apparatus based on a closed system, characterized in than it consists of connecting a closed line system (1) onto the line system of a respirating apparatus, which is provided with means (pump (4),atomizer (5)) by which a disinfecting means may be brought into and through the line system of the respirating apparatus.

## Description

The invention relates to a device and a method for the disinfection of a respirating apparatus for patients, more in particular of a respirating or anaesthesia apparatus based on a closed system.

The disinfection of the line system of a respirating apparatus, i.e. of the gas-carrying parts of the apparatus, at present takes place by means of disassembling the parts from the apparatus which are to be disinfected, and the immersion of those parts in a bath containing the disinfecting means, and the replacement of the parts which are meant for once-only use.
For a completely closed respirating system such as is known from EP-A-0 281 180, this way of disinfecting is considered difficult, because of the nature of the construction of this respirating system, which does not enable the parts which are to be disinfected, to be disconnected or taken out easely, and to be mounted back in again after the disinfection.
For this reason, the disinfection of the closed respirating system in the manner as presently applied, is very complicated.

The invention aims to obviate this drawback of the known method of disinfection.
The device according to the invention is to that end characterized in that it consists of a closed line system to be connected onto the line system of a closed respirating apparatus, which is provided with means by which a disinfecting means may be carried through the line system of the respirating apparatus.
The invention further relates to a method for the disinfection of a respirating apparatus, characterized in that a disinfecting means is carried through the line system of the respirating apparatus from a closed line system which is connected onto the line system of the respirating apparatus.

The disinfecting substances are thereby only present within the line system in such a way that, contrary to the known method of disinfection, the disinfecting substances do not contact the open air.

According to a characteristic of the invention, the line system is provided with means for supplying the disinfecting means directly into the line system.

These means may consist of an atomizer by means of which an aerosol is prepared. This aerosol may be composed of peracetic acid.
A circulating pump may further be included in the line system, by means of which the disinfecting means may be carried through the closed line system via the connection thereon into the line system of the respirating apparatus, and there be circulated, whereby the disinfecting means is carried from the line system of the respirating apparatus via an outlet into the closed line system of the device.

The line system is further provided with means by which the disinfecting means, which are circulated through the respirating apparatus may be withdrawn and neutralized.

With use of peracetic acid aerosol this may take place by means of natriumthiosulfate.

The line system may further be provided with different connections for the supply of other means to be circulated.

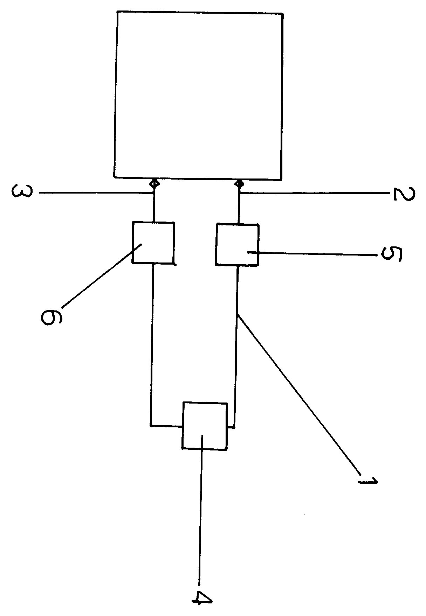

The invention will now be explained more closely with reference to the drawing by way of example.

As shown in the drawing, the device consists of a closed line system 1 with an inlet 2 to and into the line system of the respirating apparatus, and an outlet 3 from the line system of the respirating apparatus to and into the closed line system of the device according to the invention. Both the line systems, as well as the one according to the invention as the one of the respirating apparatus are during use connected onto each other leakproof.

A circulating pump 4 is included in the line system by means of which the disinfecting means is circulated within the line systems which are connected onto each other. The line system is further provided with an atomizer or spray 5 into which the disinfecting means is supplied and in which it is formed into an aerosol and delivered into the line system. The aerosol, e.g. peracetic acid is brought into and through the line system of the respirating apparatus and returns in the closed line system of the device, where the means is withdrawn by means of for example a filter 6 and neutralized for example by natriumthiosulfate.

After the disinfecting, other means may be circulated through the line system, for the neutralizing whether mechanically removing of residue of the disinfecting means, to be found in the line systems.

## Claims

1. Device for the disinfecting of the line system of a respirating apparatus for patients, more in particular of a respirating apparatus based on a closed system, characterized in that it consists of a closed line system, to be connected onto the line system of a respirating apparatus, which is provided with means by which the disinfecting means may be carried in and through the line system of the respirating apparatus.

2. Device according to claim 1, characterized in that the line system includes a circulating pump.

3. Device according to claim 1 or 2, characterized in that means are present for supplying the disinfecting means directly into the line system.

4. Device according to claim 3, characterized in that the means for supplying the disinfecting means directly into the line system are formed by an atomizer.

5. Device according to one of the preceding claims, characterized in that it is provided with means for neutralizing and withdrawing of the disinfecting means.

6. Method for the disinfecting of a respirating apparatus, more in particular of a respirating apparatus based on a closed system, characterized in that the disinfecting means is supplied directly through the line system of the respirating apparatus from a closed line system connected thereon.

7. Method according to claim 6, characterized in that the disinfecting means is in the form of an aerosol.

8. Method according to claim 7, characterized in that the disinfecting means is atomized within the closed line system.

9. Method according to one of the claims 6 to 8, characterized in that the disinfecting means consists of peracetic acid.
